# EUROPEAN PATENT SPECIFICATION

(11) **EP 3 474 286 B1**
(45) Date of publication and mention of the grant of the patent: **25.09.2024**
(21) Application number: 18200665.0
(22) Date of filing: 16.10.2018
(51) Int. Cl.: A61G 15/02, A61G 15/16, G16H 40/60, G05B 19/042

(54) **MEDICAL CARE APPARATUS AND MEDICAL CARE SYSTEM**
VORRICHTUNG ZUR MEDIZINISCHEN PFLEGE UND SYSTEM ZUR MEDIZINISCHEN PFLEGE
APPAREIL ET SYSTÈME DE SOINS MÉDICAUX

(30) Priority: 18.10.2017 JP 2017201788
(43) Date of publication of application: 24.04.2019
(73) Proprietor: J. MORITA TOKYO MFG. CORP., Kitaadachi-gun Saitama 362-0806 (JP)
(72) Inventor: SONOBE, Kouichi, Kyoto-shi, Kyoto, 612-8533 (JP); ITO, Tetsuzo, Kyoto-shi, Kyoto, 612-8533 (JP)
(74) Representative: Müller Hoffmann & Partner

(56) References cited:
- EP-A1- 0 697 661
- US-A- 5 190 349
- US-B1- 6 470 222

## Description

### BACKGROUND OF THE INVENTION

### Field of the Invention

The present invention relates to a medical care apparatus and a medical care system.

### Description of the Background Art

For determining occurrence of a failure and predicting a failure, a medical care apparatus such as a chair unit and an X-ray apparatus that are installed in a hospital or a clinic is configured to include a sensor and collect the results detected by the sensor as history information (WO 2014/042057).

Specifically, in a medical apparatus disclosed in WO 2014/042057, a temperature sensor is provided in the vicinity of a power device such as a converter and an inverter configured to generate a high voltage to be supplied to an X-ray tube, so as to collect the data, as history information, showing the temperature change obtained by the temperature sensor. In the medical apparatus disclosed in WO 2014/042057, occurrence of a failure is determined or predicted based on the collected history information.

### SUMMARY OF THE INVENTION

For the medical apparatus disclosed in WO 2014/042057, it is necessary to separately provide a sensor for collecting history information and a configuration for processing the result detected by the sensor. Thus, the existing board needs to be improved. Furthermore, in the medical apparatus disclosed in WO 2014/042057, all of the results detected by the sensor are stored as history information. Accordingly, when the apparatus becomes complicated so that the number of sensors is significantly increased, there occurs a problem that the amount of the history information to be stored dramatically increases.

Further medical care apparatuses are known from US 5,190,349, US 6,470,222, and EP 0 697 661 A1.

The present invention has been made in order to solve the above-described problems. An object of the present invention is to provide a medical care apparatus and a medical care system, for which history information can be collected without modifying the existing board and the system configuration, and the amount of history information to be stored can be suppressed.

The object is solved by a medical care apparatus according to claim 1 and a medical care system according to the further independent claim. Preferred embodiments are defined in the dependent claims.

A medical care apparatus according to the present invention includes: a plurality of control boards configured to communicate with one another and perform a plurality of types of control operations; and a history collection board connected to the plurality of control boards so as to be capable of communicating with the plurality of control boards, the history collection board being configured to collect history information from each of the plurality of control boards. The history collection board includes a collection unit configured to collect history information about each of the plurality of types of control operations from communication data that is transmitted and received among the plurality of control boards, and a storage unit configured to store the history information collected by the collection unit. The collection unit is configured to collect history information that changes from the history information previously stored in the storage unit and cause the storage unit to store the history information collected.

A medical care system according to the present invention includes: the above-described medical care apparatus; and an analysis apparatus configured to analyze history information stored in the medical care apparatus.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic diagram for illustrating the configuration of a medical care system according to the first embodiment of the present invention.
Fig. 2 is a block diagram for illustrating the configuration of a medical care apparatus according to the first embodiment of the present invention.
Fig. 3 is a block diagram for illustrating the configuration of an analyzing computer according to the first embodiment of the present invention.
Fig. 4 is a diagram for illustrating the contents of communication packets that are transmitted and received among control boards.
Fig. 5 is a diagram for illustrating history information collected by a history collection unit according to the first embodiment of the present invention.
Fig. 6 is a diagram for illustrating: the timing at which the history collection unit according to the first embodiment of the present invention collects history information; and an analysis result thereof.
Fig. 7 is a schematic diagram for illustrating a system to which a plurality of medical care systems are connected.

### DESCRIPTION OF THE PREFERRED EMBODIMENTS

### (First Embodiment)

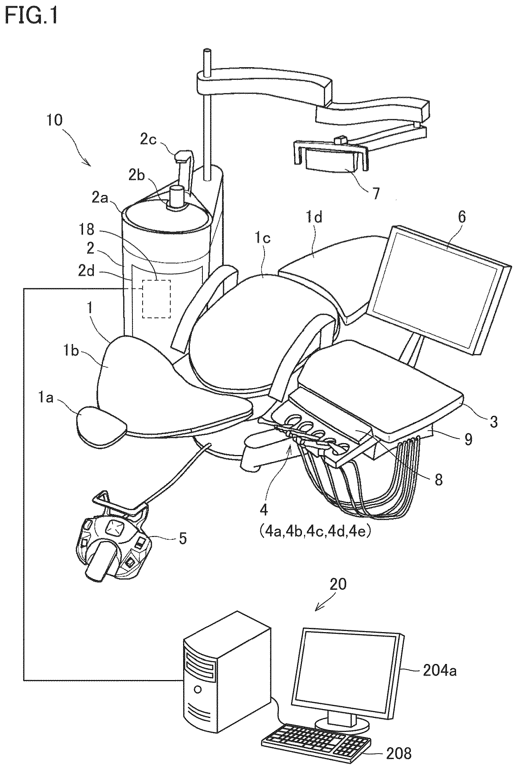

Fig. 1 is a schematic diagram for illustrating the configuration of a medical care system according to the first embodiment of the present invention. The medical care system shown in Fig. 1 consists of: one medical care apparatus (which will be hereinafter also referred to as a care apparatus) 10 serving as a chair unit; and one analyzing computer 20. It is to be noted that a plurality of medical care apparatuses 10 may be connected to one analyzing computer 20.

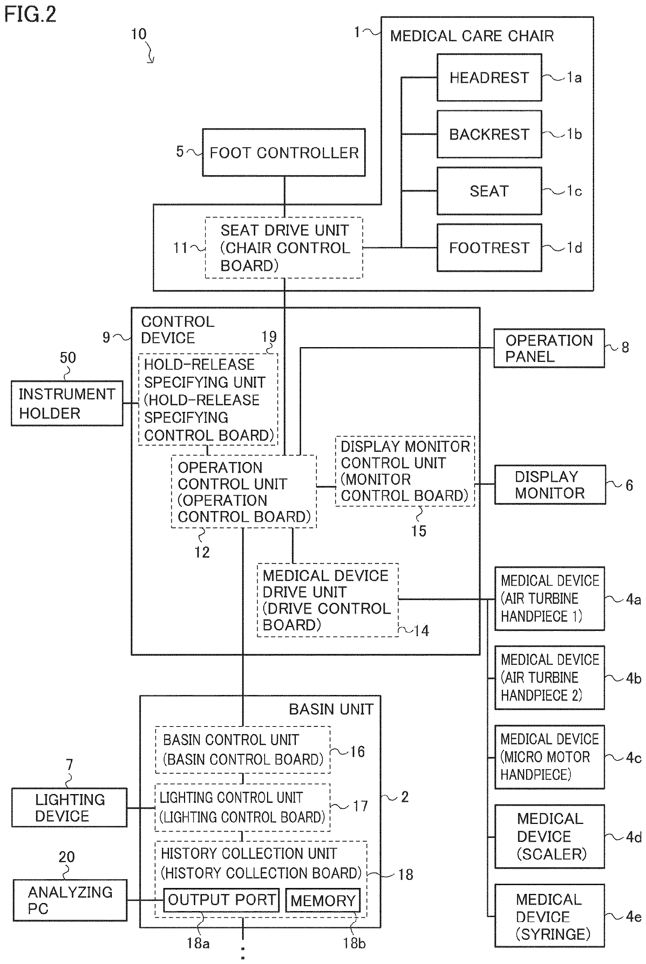

First, medical care apparatus 10 will be hereinafter described in detail. Fig. 2 is a block diagram for illustrating the configuration of a medical care apparatus according to the first embodiment of the present invention. As shown in Figs. 1 and 2, medical care apparatus 10 includes a medical care chair 1, a foot controller 5, a tray table 3, a control device 9, an instrument holder 50, an operation panel 8, a display monitor 6, a basin unit 2, and a lighting device (operation light) 7.

Medical care chair 1 includes a headrest 1a supporting the head of a patient, a backrest 1b supporting the back of the patient, a seat 1c supporting buttocks of the patient, a footrest 1d supporting legs of the patient, and a seat drive unit 11. Seat drive unit 11 is formed of a central processing unit (CPU), a read only memory (ROM), a random access memory (RAM) and the like that are mounted on a chair control board, and configured to control driving of each of headrest 1a, backrest 1b, seat 1c, and footrest 1d.

For example, seat 1c is raised or lowered under the control of seat drive unit 11. Each of headrest 1a, backrest 1b and footrest 1d is moved under the control of seat drive unit 11 in the direction that is perpendicular or horizontal to seat 1c. When headrest 1a, backrest 1b and footrest 1d are moved in the direction perpendicular to seat 1c, the patient sitting on medical care chair 1 is brought into a seated posture. When headrest 1a, backrest 1b and footrest 1d are moved in the direction horizontal to seat 1c, the patient sitting on medical care chair 1 is laid on his/her back. In this way, seat drive unit 11 drives headrest 1a, backrest 1b, seat 1c and footrest 1d to thereby change the posture of medical care chair 1.

Foot controller 5 has a plurality of switches (pedals) configured to receive the pressing-down operation by the user's foot. The user can allocate a prescribed function to each of the plurality of switches.

For example, the user can allocate the function of changing the posture of medical care chair 1 to a switch on foot controller 5. When the user uses his/her foot to press down the switch to which the function of changing the posture of medical care chair 1 is allocated, foot controller 5 outputs a control signal to seat drive unit 11. Based on the control signal, seat drive unit 11 drives headrest 1a, backrest 1b, seat 1c, and footrest 1d.

Furthermore, the user can allocate the function of driving medical device 4 to a switch on foot controller 5. When the user uses his/her foot to press down the switch to which the function of driving medical device 4 is allocated, foot controller 5 outputs a control signal to medical device drive unit 14. Based on the control signal, medical device drive unit 14 controls driving of medical device 4.

Other functions such as functions of controlling a lighting device 7 to be turned on and off can be allocated to switches on foot controller 5.

Tray table 3 is used as a table on which tools and the like are placed during the medical care. Tray table 3 is connected to an arm (not shown) extending from medical care chair 1 or a floor. Accordingly, the user can manually move tray table 3 pivotally, horizontally and vertically relative to medical care chair 1.

Control device 9 is provided on the bottom surface of tray table 3. To this control device 9, medical care chair 1, a plurality of medical devices 4, instrument holder 50, basin unit 2, operation panel 8, and display monitor 6 are connected. Control device 9 includes an operation control unit 12, a medical device drive unit 14, a display monitor control unit 15, and a hold-release specifying unit 19, which each are formed of a CPU, a ROM, a RAM and the like that are mounted on an operation control board, a drive control board, a monitor control board, and a hold-release specifying control board, respectively.

Medical device 4 is, for example, an instrument for dental care such as an air turbine handpiece, a micro motor handpiece, a scaler, a three-way syringe, and a vacuum syringe. Medical device 4 is not limited to the above components, but may be an intraoral camera, a photopolymerization irradiator, a root canal length measuring instrument, a three-dimensional scanner and a root canal enlargement instrument, and the like, or may be a non-driving instrument such as a dental mirror, a syringe, and a filling tool.

In medical care apparatus 10, five types of medical devices 4a, 4b, 4c, 4d, and 4e are used. For example, medical devices 4a and 4b each are an air turbine handpiece. Medical device 4c is a micro motor handpiece. Medical device 4d is a scaler. Medical device 4e is a three-way syringe.

Each medical device 4 is held by instrument holder 50. When medical device 4 is removed from instrument holder 50, the held state by instrument holder 50 is released, which leads to a state where medical device 4 is selected (referred to as a selected state).

Each medical device 4 is connected to medical device drive unit 14 included in control device 9. Medical device drive unit 14 drives each medical device 4 based on the user's operation of pressing down foot controller 5. For example, when the user uses his/her foot to press down the switch for driving the air turbine handpiece in foot controller 5, medical device drive unit 14 rotates a cutting tool held at a head portion of the air turbine handpiece. In this way, medical device drive unit 14 drives medical device 4 based on the user's operation performed on foot controller 5.

Instrument holder 50 serves as a holding member for holding each medical device 4. Instrument holder 50 is connected to operation control unit 12 via hold-release specifying unit 19 included in control device 9. Hold-release specifying unit 19 specifies that the state in which instrument holder 50 holds medical device 4 has been released, and then, outputs a signal showing the specified result to operation control unit 12. Based on the signal from hold-release specifying unit 19, operation control unit 12 specifies medical device 4 in the selected state.

As shown in Fig. 1, basin unit 2 is provided on the side portion of medical care chair 1. Basin unit 2 includes a bowl 2a provided with an exhaust port, a cup stand 2b on which a cup is placed, and a water faucet 2c through which water is supplied to the cup. The patient can gargle with water supplied into the cup from water faucet 2c.

As shown in Fig. 2, basin unit 2 includes a basin control unit 16. Basin control unit 16 is configured to control the flow of water that is used in medical care apparatus 10. Basin control unit 16 is formed of a CPU, a ROM, a RAM, and the like that are mounted on a basin control board.

Furthermore, basin unit 2 includes a lighting control unit 17. Lighting control unit 17 is configured to control lighting device 7 to be turned on and off. Lighting control unit 17 is formed of a CPU, a ROM, a RAM, and the like that are mounted on an illumination control board.

Furthermore, basin unit 2 includes a history collection unit 18 configured to collect the history information in medical care apparatus 10. History collection unit 18 is formed of a CPU, a ROM, a RAM, and the like that are mounted on a history collection board. Also, history collection unit 18 is provided inside basin unit 2 that is closed by a maintenance door 2d. Thus, memory 18b cannot be removed from the history collection board unless maintenance door 2d is opened using a prescribed tool. It is to be noted that history collection unit 18 corresponds to one embodiment of the "collection unit". Also, memory 18b corresponds to one embodiment of the "storage unit".

The history collection board is provided with an output port 18a through which the history information is output to analyzing computer 20. Thus, a maintenance worker opens maintenance door 2d to connect a wiring line extending from analyzing computer 20 to output port 18a, thereby allowing the collected history information to be extracted. Alternatively, analyzing computer 20 and history collection unit 18 in medical care apparatus 10 may be continuously connected to each other through output port 18a.

The history information collected by history collection unit 18 is stored in memory 18b that is attachable to and detachable from the history collection board. Memory 18b is a storage medium formed of a nonvolatile memory such as a memory card. Memory 18b can be removed from the history collection board when a maintenance worker opens maintenance door 2d. Accordingly, the wiring line extending from analyzing computer 20 does not have to be connected to output port 18a, but memory 18b only has to be removed from the history collection board and connected to analyzing computer 20, so that the collected history information can be extracted. In addition, output port 18a does not have to be provided in the history collection board. When output port 18a is not provided in the history collection board, the history information is to be extracted using only memory 18b from medical care apparatus 10 and transferred to analyzing computer 20.

For communication between the control boards inside medical care apparatus 10, relevant known techniques such as CAN (Controller Area Network) communication are applied. In medical care apparatus 10, all of the control boards such as the chair control board, the operation control board, the drive control board, the monitor control board, the hold-release specifying control board, the basin control board, and the illumination control board communicate with one another through CAN communication, so as to share each other's situations for performing control operations. In CAN communication, communication is established among the control boards through transmission of communication packets carrying communication data that includes the information about the control operation by each control board. For the communication among the control boards, if the communication data including the information about the control operation by each control board is transmitted and received among the control boards, it is not necessary to use communication packets for this transmission and reception. In this case, the information about the control operation includes a plurality of pieces of information about a switch, a sensor, an actuator, a status and the like that are controlled by their respective control boards.

When the history collection board is connected to other control boards so as to be capable of CAN communication with these other control boards, history collection unit 18 can collect the information about the control operation by each control board as history information. For example, through CAN communication, history collection unit 18 collects the information about various types of control operations as history information from the communication packets including: the information about the chair unit position control transmitted from seat drive unit 11; the information about medical device 4 transmitted from operation control unit 12; the information about the cup water supply electromagnetic valve control transmitted from basin control unit 16; and the information about the lighting control of lighting device 7 transmitted from lighting control unit 17. In addition, the history information collected by history collection unit 18 includes at least the operation history about the operation performed by medical care apparatus 10, and the detection history detected by medical care apparatus 10.

Display monitor 6 is provided at an arm extending upward from tray table 3, as shown in Fig. 1. Display monitor 6 has a screen with a large display area. In other words, display monitor 6 can display a great amount of information.

Display monitor 6 is connected to operation control unit 12 through display monitor control unit 15 provided in control device 9. Based on the command from operation control unit 12, display monitor control unit 15 causes display monitor 6 to display an image, a related image related to the operation image, and the like.

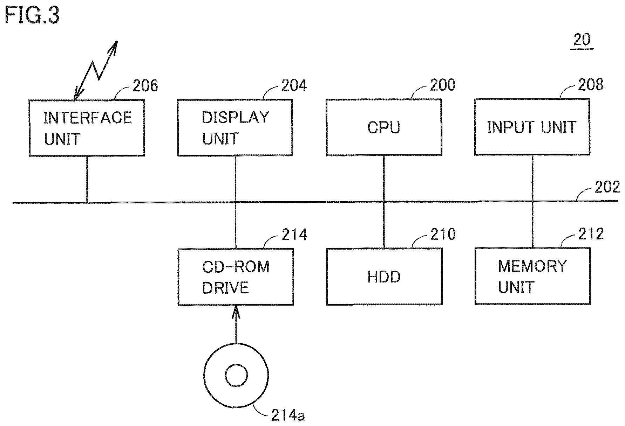

Then, analyzing computer 20 will be hereinafter described in detail. Fig. 3 is a block diagram for illustrating the configuration of analyzing computer 20 according to the first embodiment of the present invention. Analyzing computer 20 includes: a CPU 200 configured to execute various programs including an operating system (OS); a memory unit 212 in which the data required for executing the program by CPU 200 is temporarily stored; and a hard disk drive (HDD) 210 in which the program executed by CPU 200 is stored in a non-volatile manner. Also, the program for implementing analysis is stored in advance in hard disk drive 210, and read by a compact disk-read only memory (CD-ROM) drive 214 and the like from a storage medium such as a CD-ROM 214a.

CPU 200 receives instructions from the user and the like through an input unit 208 formed of a keyboard, a mouse or the like, and outputs the analysis result and the like obtained as a result of execution of the program to a display device 204a through a display output unit 204. These units are connected to one another through a bus 202. Furthermore, an interface unit 206 can be connected to output port 18a of medical care apparatus 10. In the above description, analyzing computer 20 and medical care apparatus 10 are connected in a wired manner through output port 18a, but may be connected wirelessly.

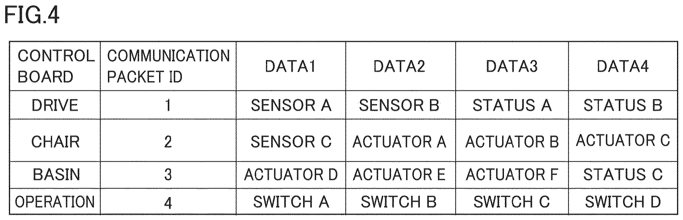

Then, a detailed explanation will be given with regard to the process in which history collection unit 18 collects history information from each control board in medical care apparatus 10 according to the present embodiment. Fig. 4 is a diagram for illustrating the contents of communication packets that are transmitted and received among the control boards. It is to be noted that the contents of the communication packets shown in Fig. 4 are shown merely by way of example, and other types of data are also allocated to communication packets.

Each control board performs CAN communication, in which communication is established through communication packets carrying a plurality of pieces of information about the switch, the sensor, the actuator, the status and the like that are controlled by their respective control boards (the information about control). For all of the control boards provided in medical care apparatus 10, the formats for the communication packets are defined in advance such that each information about the switch, the sensor, the actuator, the status and the like controlled by their respective control boards can be identified. Specifically, for the communication packets shown in Fig. 4, a communication packet ID "1" is allocated to the drive control board (medical device drive unit 14); a communication packet ID "2" is allocated to the chair control board (seat drive unit 11); a communication packet ID "3" is allocated to the basin control board (basin control unit 16); and a communication packet ID "4" is allocated to the operation control board (operation control unit 12).

For the communication packet allocated with communication packet ID " 1", and regarding medical device drive unit 14 controlling a sensor A, a sensor B, a status A, and a status B, (i) the information about sensor A is allocated to "DATA1", (ii) the information about sensor B is allocated to "DATA2", (iii) the information about status A is allocated to "DATA3", and (iv) the information about status B is allocated to "DATA4". Sensor A serves, for example, to detect the drive signal for medical device 4a of the air turbine handpiece controlled by medical device drive unit 14. Sensor B serves, for example, to detect the drive signal for medical device 4c of the micro motor handpiece controlled by medical device drive unit 14. Status A indicates, for example, the information showing the driving mode of medical device 4a driven by medical device drive unit 14. Status B indicates, for example, the information showing the driving mode of medical device 4c driven by medical device drive unit 14.

For the communication packet allocated with communication packet ID "2", and regarding seat drive unit 11 controlling a sensor C, an actuator A, an actuator B, and an actuator C, (i) the information about sensor C is allocated to "DATA1", (ii) the information about actuator A is allocated to "DATA2", (iii) the information about actuator B is allocated to "DATA3", and (iv) the information about actuator C is allocated to "DATA4". Sensor C serves, for example, to detect abnormalities in the actuator provided in medical care chair 1. Actuator A is, for example, provided in headrest 1a of medical care chair 1. Actuator B is, for example, provided in backrest 1b of medical care chair 1. Actuator C is, for example, provided in seat 1c of medical care chair 1.

Similarly, for the communication packet allocated with communication packet ID "3", and regarding basin control unit 16 controlling an actuator D, an actuator E, an actuator F, and a status C, (i) the information about actuator D is allocated to "DATA1", (ii) the information about actuator E is allocated to "DATA2", (iii) the information about actuator F is allocated to "DATA3", and (iv) the information about status C is allocated to "DATA4". For the communication packet allocated with communication packet ID "4", and regarding operation control unit 12 controlling a switch A, a switch B, a switch C, and a switch D, (i) the information about switch A is allocated to "DATA1", (ii) the information about switch B is allocated to "DATA2", (iii) the information about switch C is allocated to "DATA3", and (iv) the information about switch D is allocated to "DATA4".

Each control board transmits the communication packets (shown in Fig. 4) carrying each information about the switch, the sensor, the actuator, the status and the like to other control boards, so that all of the control boards can operate in cooperation with one another. Thus, the history collection board is connected to the wiring line capable of receiving a communication packet, so that history collection unit 18 can collect, from a plurality of control boards, a plurality of pieces of information about the control operations by the plurality of control boards as a plurality of pieces of history information.

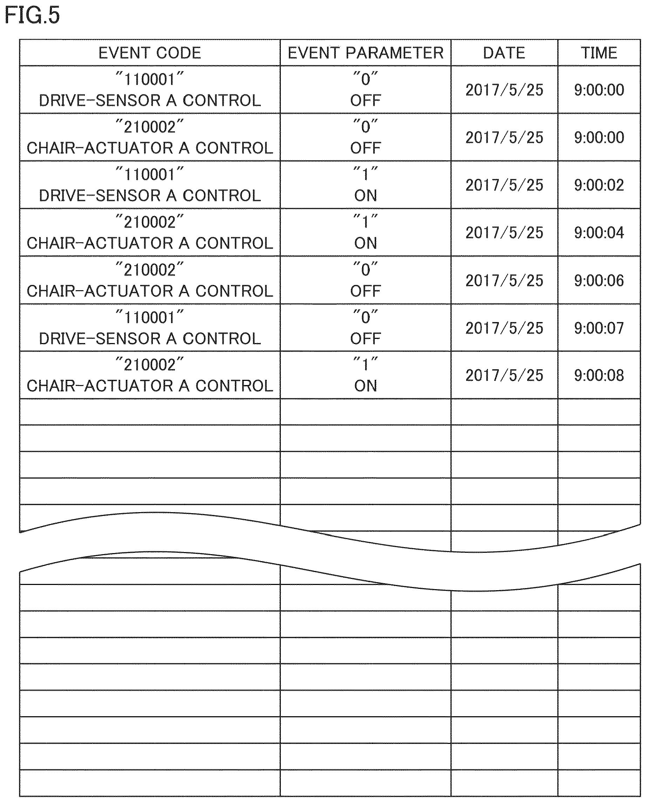

Fig. 5 is a diagram for illustrating history information collected by history collection unit 18 according to the first embodiment of the present invention. For the communication packet that is transmitted and received among the control boards, the information is identified by an event code attached according to the format shown in Fig. 4. For example, when the communication packet includes the information about sensor A ("DATA1") of the drive control board (communication packet ID "1"), history collection unit 18 generates communication packet ID "1" showing information for identifying the drive control board and an event code "110001" corresponding to the information for identifying sensor A, and then, stores the generated ID and code as history information. Accordingly, the communication packet ID and the event code that corresponds to the information for identifying the sensor and the like are stored without fail as the history information collected by history collection unit 18. Thus, it becomes possible to identify as to which control board the information indicates, as to which sensor the information indicates, and the like. Specifically, "110002", "110003" and "110004" are allocated as event codes corresponding to sensor B ("DATA2"), status A ("DATA3") and status B ("DATA4"), respectively, of the drive control board (communication packet ID "1"). Furthermore, "210001", "210002", "210003", and "210004" are allocated as event codes corresponding to sensor C ("DATA1"), actuator A ("DATA2"), actuator B ("DATA3"), and actuator C ("DATA4"), respectively, of the chair control board (communication packet ID "2").

Furthermore, history collection unit 18 generates an event parameter corresponding to the data included in the communication packet, and stores the generated event parameter as history information. For example, when the communication packet includes the information showing that sensor A ("DATA1") of the drive control board is in an OFF state, history collection unit 18 generates an event parameter "0" corresponding to the information "DATA1"showing that sensor A is in an OFF state, and stores the generated event parameter as history information. As the history information collected by history collection unit 18, the information about date, time and the like is also stored together with the event codes and the event parameters. In history collection unit 18, not all of the communication packets are stored as will be described later. Instead, when there are time-dependent changes in communication packet ID and data that are included in the communication packet (when an event occurs), an event code and an event parameter are generated and stored as history information.

Specifically, as history information shown in Fig. 5, the information of the event code "110001" with the event parameter of "0" is stored in memory 18b (see Fig. 2) together with the information about the date (2017/5/25) and the time (9:00:00). Similarly, as history information shown in Fig. 5, the information of the event code "210002" with the event parameter of "0" is stored in memory 18b (see Fig. 2) together with the information about the date (2017/5/25) and the time (9:00:00). In this case, the event code "210002" shows actuator A ("DATA2") of the chair control board (communication packet ID "2"), and the event parameter "0" shows that actuator A is in an OFF state.

In CAN communication, even when the subject controlled by the control board does not change, the communication packet carrying information is transmitted to other control boards. Specifically, when medical device 4a of the air turbine handpiece is not operated, the communication packet carrying the information showing that sensor A of the drive control board is in the OFF state is continuously transmitted to other control boards. Thus, when every information showing that sensor A is in the OFF state is collected by history collection unit 18 from the communication packets transmitted from the drive control board, and the collected information is stored in memory 18b, then, the amount of information to be stored enormously increases to exceed the capacity that can be stored in memory 18b in a short period of time.

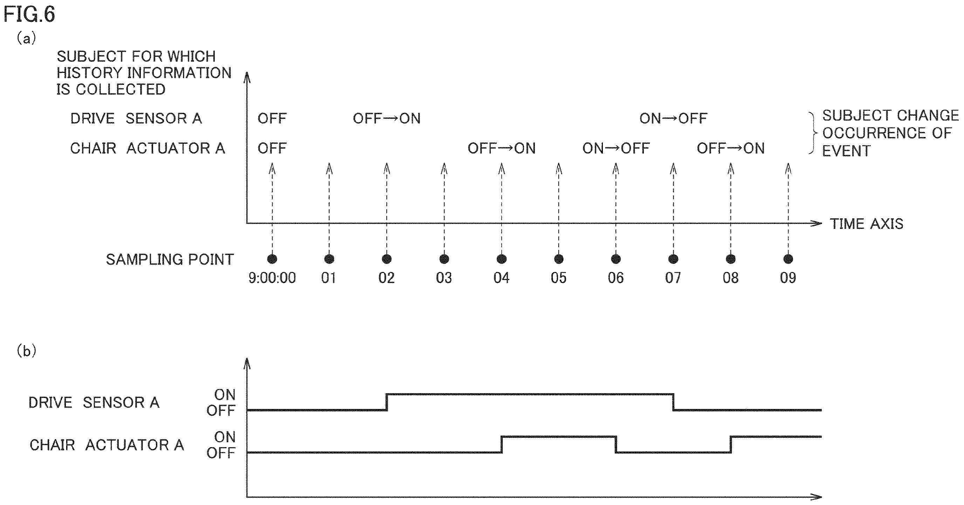

Thus, in history collection unit 18 according to the present embodiment, when the subject controlled by the control board changes (when an event occurs), history information is collected from the communication packets. Fig. 6 is a diagram for illustrating: the timing at which history collection unit 18 according to the first embodiment of the present invention collects history information; and an analysis result thereof. Fig. 6(a) shows: sensor A ("DATA1") of the drive control board (communication packet ID "1"); and actuator A ("DATA2") of the chair control board (communication packet ID "2"), both of which are subjects for which history information is collected. Furthermore, history collection unit 18 collects history information from the communication packets at a sampling point at intervals of one second.

History collection unit 18 starts to collect the history information from time (9:00:00) on date (2017/5/25). First, at time (9:00:00), history collection unit 18 collects: the information showing that sensor A is in the OFF state; and the information showing that actuator A is in the OFF state. Then, history collection unit 18 stores the history information in memory 18b, as shown in Fig. 5.

Then, history collection unit 18 collects the information at time (9:00:01). In this case, however, since sensor A remains in the OFF state and actuator A remains in the OFF state, the collected information is not stored as history information in memory 18b, as shown in Fig. 5.

Then, history collection unit 18 collects the information at time (9:00:02). In this case, since sensor A has changed into the ON state, the history information is stored in memory 18b, as shown in Fig. 5. Specifically, history collection unit 18 causes memory 18b to store the information of the event code" 110001" with the event parameter of "1" together with the information of the date (2017/5/25) and the time (9:00:02). In addition, since actuator A remains in the OFF state, history collection unit 18 does not cause memory 18b to store the history information about actuator A, as shown in Fig. 5. History collection unit 18 performs the similar process also for the time (9:00:07).

History collection unit 18 collects information at time (9:00:03, 9:00:05, and 9:00:09). However, in this case, since sensor A remains in the OFF state and actuator A remains in the OFF state, the collected information is not stored as history information in memory 18b, as shown in Fig. 5.

History collection unit 18 collects information at time (9:00:04 and 9:00:08). In this case, since actuator A has changed into the ON state, the history information is stored in memory 18b, as shown in Fig. 5. Specifically, history collection unit 18 causes memory 18b to store the information of the event code "210002" with the event parameter of "1" together with the information of the date (2017/5/25) and the time (9:00:04 and 9:00:08). In addition, since sensor A remains in the OFF state, history collection unit 18 does not cause memory 18b to store the history information about sensor A, as shown in Fig. 5.

History collection unit 18 collects information at time (9:00:06). In this case, since actuator A has changed into the OFF state, the history information is stored in memory 18b, as shown in Fig. 5. Specifically, history collection unit 18 causes memory 18b to store the information of the event code "210002" with the event parameter of "0" together with the information of the date (2017/5/25) and the time (9:00:06). In addition, since sensor A remains in the OFF state, history collection unit 18 does not cause memory 18b to store the history information about sensor A, as shown in Fig. 5.

As having been described with reference to Fig. 6(a), history collection unit 18 collects, from the communication packets, the history information that has changed from the previously stored history information, and then causes memory 18b to store the collected history information. Accordingly, the history information stored in memory 18b can be reduced in amount as shown in Fig. 5, which eliminates the need to prepare memory 18b with a large capacity. Even when analyzing computer 20 is connected to output port 18a of the history collection board so as to extract the history information stored in memory 18b, but when the amount of the history information stored in memory 18b is relatively small, analyzing computer 20 can extract the history information stored in memory 18b in a short period of time.

Analyzing computer 20 analyzes the history information extracted from memory 18b. For example, by the history information shown in Fig. 5, it is difficult to understand how the states of sensor A and actuator A have changed. Thus, analyzing computer 20 plots a graph shown in Fig. 6(b) to show the temporal change of the history information shown in Fig. 5. In the graph shown in Fig. 6(b), it can be recognized at a glance that sensor A is in the ON state from time (9:00:02) to time (9:00:07), and, during this time period, actuator A is in the ON state from time (9:00:04) to time (9:00:06).

Since analyzing computer 20 includes display device 204a, it can cause display device 204a to display the history information stored in medical care apparatus 10 as a time series chart as shown in Fig. 6(b). By displaying the time series chart of the history information on display device 204a, the temporal change of the history information in medical care apparatus 10 can be readily grasped.

As described above, the medical care apparatus (medical care apparatus 10) according to the present first embodiment collects the history information about various types of control operations from the communication packets that are transmitted and received among a plurality of control boards. Accordingly, the history information can be collected without modifying the existing board and the system configuration. Particularly, the history collection board merely receives and monitors the communication packet, which does not influence the control over the entire apparatus. In other words, the algorithm for collecting the history information does not influence the system of the apparatus at all, so that the existing apparatus can be readily modified into an apparatus capable of collecting history information. Furthermore, in the medical care apparatus according to the present first embodiment, the history information that has changed from the previously stored history information is stored, so that the amount of the history information to be stored can be reduced.

The medical care system according to the present first embodiment includes analyzing computer 20 configured to analyze the history information, so that the history information collected in medical care apparatus 10 can be analyzed. Specifically, analyzing computer 20 plots a time series chart of the history information, so that the history information collected in medical care apparatus 10 can be rendered visible.

### (Second Embodiment)

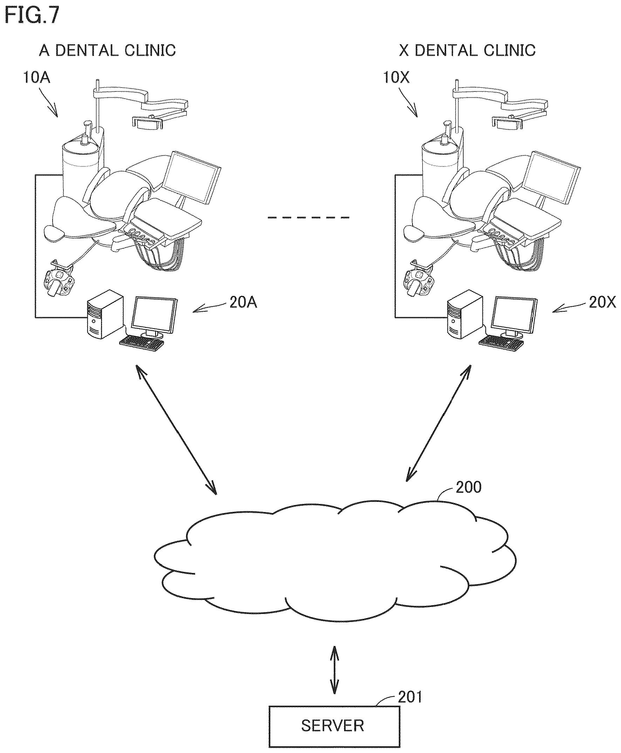

In the medical care system according to the present first embodiment, the history information of medical care apparatus 10 installed in a clinic is merely collected and analyzed. However, at least one of the collected history information of medical care apparatus 10 and the analysis result thereof can be shared in common among a plurality of medical care apparatuses provided in the clinic. Furthermore, at least one of a plurality of pieces of history information collected from a plurality of medical care apparatuses 10 installed in a plurality of clinics; and the analysis results thereof can also be shared in common among the medical care apparatuses installed in the plurality of clinics. For example, the medical care systems installed in a plurality of clinics are connected to a server on a cloud managed by the manufacturer of the medical care systems, so that the collected plurality of pieces of history information of medical care apparatuses 10 and the analysis results thereof are shared in common. Specifically, Fig. 7 is a schematic diagram for illustrating a system to which a plurality of medical care systems are connected. In the system shown in Fig. 7, an analyzing computer 20A of a medical care system installed in A dental clinic is connected to a server 201 on a cloud 200 through a network line. In this system, similarly, an analyzing computer 20X of another medical care system installed in X dental clinic is connected to server 201 on cloud 200 through the network line. In other words, in the present system, at least one of a plurality of pieces of history information about medical care apparatuses 10 collected in a plurality of medical care systems installed in dental clinics from A dental clinic to X dental clinic; and the analysis results thereof can be shared in common through server 201 on cloud 200. In addition, analyzing computer 20 provided in each clinic is connected to the network line through interface unit 206.

In server 201 on cloud 200, a plurality of pieces of history information about medical care apparatuses 10 collected from the plurality of clinics and the analysis results thereof can be summarized to calculate the overall average value, or a plurality of pieces of history information about medical care apparatuses 10 collected from a plurality of clinics in a specific area and the analysis results thereof can be summarized to calculate an average value for the specific area. Furthermore, in server 201 on cloud 200, based on the plurality of pieces of history information of medical care apparatuses 10 and the analysis results thereof, the information for improving medical care apparatus 10 can be provided to each clinic. Also, by comparing the history information of medical care apparatus 10 and the analysis results thereof between one clinic and other clinics, the information proposing methods of utilizing and managing medical care apparatus 10 can be provided.

As described above, in the medical care system according to the present second embodiment, at least one of the history information stored in medical care apparatus 10 and the analysis result thereof can be transmitted through analyzing computer 20 to server 201. In other words, analyzing computer 20 corresponds to a communication unit.

In addition, analyzing computer 20 does not necessarily have to be provided in each clinic, but there may be a communication unit (for example, a transmission server and the like) through which at least one of the history information collected from medical care apparatus 10 in each clinic and the analysis result thereof can be transmitted to server 201 on cloud 200. Furthermore, the analyses of a plurality of pieces of history information conducted by analyzing computer 20 may be collectively performed in server 201 on cloud 200.

### (Modification)

(1) Medical care apparatus 10 according to the above-mentioned embodiment has been described as a chair unit. However, the medical care apparatus may be a component other than a chair unit. Specifically, the medical care apparatus may be an X-ray apparatus, a laser treatment apparatus, and the like. The medical care apparatus such as an X-ray apparatus and a laser treatment apparatus is provided with a history collection unit that is configured to collect history information such as an operation history from the medical care apparatus such as an X-ray apparatus and a laser treatment apparatus.
(2) With regard to medical care apparatus 10 according to the above-mentioned embodiment, it has been described that history collection unit 18 is provided in each of the medical care apparatuses. However, when a plurality of medical care apparatuses 10 are provided, a specific medical care apparatus 10 may include history collection unit 18 for collecting history information from other medical care apparatuses 10. Furthermore, a history collection unit provided outside medical care apparatus 10 (for example, a history collection server and the like) may collect history information from a plurality of medical care apparatuses 10. Furthermore, the history information from the medical care apparatus such as an X-ray apparatus and a laser treatment apparatus may also be collected by a history collection unit provided in a specific medical care apparatus 10 or by a history collection unit provided outside.
(3) With regard to medical care apparatus 10 according to the above-mentioned embodiment, it has been described that history collection unit 18 is configured to collect information about each of the control operations by a plurality of control boards (for example, each information about the switch, the sensor, the actuator, the status and the like that are controlled by their respective control boards) as history information to be collected. However, history collection unit 18 may be configured to collect, as history information to be collected, only the information about a control operation selected from among various types of control operations performed by a plurality of control boards. In other words, history collection unit 18 does not collect the information about all of the control operations by the plurality of control boards as history information, but sets in advance an event code about the information to be collected, and then collects the history information about the control operation selected based on the event code. For example, history collection unit 18 sets the event codes, for which information is to be collected, as "110001" and "210002" in advance. Then, history collection unit 18 collects only event parameters with event codes of "110001" and "210002" from the communication packets, and stores the collected event parameters in memory 18b as history information.
(4) Analyzing computer 20 may output the advice information about hospital management. For example, analyzing computer 20 compares the analyzed results and the national average data, and outputs the advice information about the method of utilization a medical care apparatus, addition of a medical care apparatus, and the like for establishing clinic management.
   Analyzing computer 20 can analyze the length of time during which medical care apparatus 10 or various types of instruments are used. Thus, based on such the length of time, analyzing computer 20 can predict a failure and make a notification about the timing for replacement of a consumable part.
(5) According to the medical care system of the above-mentioned embodiment, as one exemplary embodiment, an explanation has been given with regard to a medical care system including a medical care apparatus that can be used for dental care.
However, the medical care system can be applied not only to dentistry but also to the medical examination of all medical departments such as ophthalmology, otolaryngology, radiology, and a veterinary medicine.

Although the present invention has been described and illustrated in detail, it is clearly understood that the same is by way of illustration and example only and is not to be taken by way of limitation, the scope of the present invention being defined by the terms of the appended claims.

## Claims

1. A medical care apparatus (10) comprising: a plurality of units, the plurality comprising a seat drive unit (11), and operation control unit (12),
the plurality of units being configured to communicate with one another and perform a plurality of types of control operations; and
a history collection unit (18) connected to other control units (12,14-17,19) configured to communicate with the control units (12,14-17,19), the history collection device being configured to collect usage data from each of the control units (12,14-17,19),
the history collection unit including
a collection unit(18) configured to collect usage data about each of the plurality of types of control operations from communication data that is transmitted and received among the control units (12,14-17,19), and
a storage unit (18b) configured to store the usage data collected by the collection unit (18),
the collection unit (18) being configured to collect usage data and cause the storage unit (18b) to store collect usage data that has changed with respect to usage data currently stored in the storage unit (18b).

2. The medical care apparatus (10) according to claim 1, wherein the collection unit (18) is configured to collect information about a control operation by each of the control units (12,14-17,19) as usage data to be collected.

3. The medical care apparatus (10) according to claim 1, wherein the collection unit (18) is configured to collect, as usage data to be collected, information about a control operation selected from among the plurality of types of control operations performed by the control units (12,14-17,19).

4. The medical care apparatus (10) according to any one of claims 1 to 3, wherein the history collection unit further includes an output unit (18a) configured to output the usage data stored in the storage unit (18b).

5. The medical care apparatus (10) according to any one of claims 1 to 3,
wherein the storage unit (18b) is a storage medium that is attachable to and detachable from the history collection device.

6. The medical care apparatus (10) according to claim 5, wherein the storage unit (18b) is provided inside a case included in the medical care apparatus (10) and has a door (2d) that can be opened and closed by using a prescribed tool.

7. The medical care apparatus (10) according to any one of claims 1 to 6, wherein the history collection unit is attachable to and detachable from a wiring line through which the communication data transmitted and received among the control units (12,14-17,19) can be received.

8. The medical care apparatus (10) according to any of claims 1 to 7, wherein the history collection unit is provided inside a basin unit (2) including a basin control unit (16).

9. A medical care system comprising:
the medical care apparatus (10) according to any one of claims 1 to 8; and
an analysis apparatus (20) configured to analyze history information extracted from the storage unit (18b).

10. The medical care system according to claim 9, further comprising a display device (204a) configured to display history information, wherein
the analysis apparatus (20) is configured to cause the display device (204a) to display, as a time series chart, the history information stored in the medical care apparatus (10).

11. The medical care system according to claim 9 or 10, further comprising a communication unit (205) configured to transmit, to a server, at least one of: the history information stored in the medical care apparatus (10); and an analysis result obtained as a result of an analysis performed by the analysis apparatus (20).

## Patentansprüche

1. Vorrichtung (10) zur medizinischen Versorgung, aufweisend: eine Vielzahl von Einheiten, wobei die Vielzahl eine Sitzantriebseinheit (11) und Bedienungssteuerungseinheit (12) aufweist,
wobei die Vielzahl von Einheiten dafür konfiguriert ist, miteinander zu kommunizieren und eine Vielzahl von Arten von Steuerungsoperation durchzuführen; und
eine Einheit (18) zur Erfassung der Vorgeschichte, die mit anderen Steuerungseinheiten (12, 14-17, 19) verbunden und dafür konfiguriert ist, mit den Steuerungseinheiten (12, 14-17, 19) zu kommunizieren, wobei die Einheit zur Erfassung der Vorgeschichte dafür konfiguriert ist, Nutzungsdaten von jeder der Steuerungseinheiten (12, 14-17, 19) zu erfassen,
wobei die Einheit zur Erfassung der Vorgeschichte umfasst:
eine Erfassungseinheit (18), die dafür konfiguriert ist, Nutzungsdaten über jede der Vielzahl von Arten von Steuerungsoperationen aus Kommunikationsdaten zu erfassen, die unter den Steuerungseinheiten (12, 14-17, 19) gesendet und empfangen werden, und
eine Speichereinheit (18b), die dafür konfiguriert ist, die mittels der Erfassungseinheit (18) erfassten Nutzungsdaten zu speichern,
wobei die Erfassungseinheit (18) dafür konfiguriert ist, Nutzungsdaten zu erfassen und zu veranlassen, dass die Speichereinheit (18b) erfasste Nutzungsdaten speichert, die sich in Bezug auf in der Speichereinheit (18b) gegenwärtig gespeicherte Nutzungsdaten geändert haben.

2. Vorrichtung (10) zur medizinischen Versorgung nach Anspruch 1, wobei die Erfassungseinheit (18) dafür konfiguriert ist, Informationen über eine Steuerungsoperation mittels jeder der Steuerungseinheiten (12, 14-17, 19) als zu erfassende Nutzungsdaten zu erfassen.

3. Vorrichtung (10) zur medizinischen Versorgung nach Anspruch 1, wobei die Erfassungseinheit (18) dafür konfiguriert ist, als zu erfassende Nutzungsdaten Informationen über eine Steuerungsoperation zu erfassen, die aus der Vielzahl von Arten von mittels der Steuerungseinheiten (12, 14-17, 19) durchgeführten Steuerungsoperationen ausgewählt wird.

4. Vorrichtung (10) zur medizinischen Versorgung nach einem der Ansprüche 1 bis 3, wobei die Einheit zur Erfassung der Vorgeschichte ferner eine Ausgabeeinheit (18a) enthält, die dafür konfiguriert ist, die in der Speichereinheit (18b) gespeicherten Nutzungsdaten auszugeben.

5. Vorrichtung (10) zur medizinischen Versorgung nach einem der Ansprüche 1 bis 3, wobei die Speichereinheit (18b) ein Speichermedium ist, das an der Einheit zur Erfassung der Vorgeschichte angebracht und aus ihr entnommen werden kann.

6. Vorrichtung (10) zur medizinischen Versorgung nach Anspruch 5, wobei die Speichereinheit (18b) innerhalb eines Gehäuses vorgesehen ist, das in der Vorrichtung (10) zur medizinischen Versorgung enthalten ist und eine Tür (2d) aufweist, die unter Verwendung eines vorgeschriebenen Instruments geöffnet und geschlossen werden kann.

7. Vorrichtung (10) zur medizinischen Versorgung nach einem der Ansprüche 1 bis 6, wobei die Einheit zur Erfassung der Vorgeschichte an einer Verdrahtungsleitung, über die die unter den Steuerungseinheiten (12, 14-17, 19) gesendeten und empfangenen Kommunikationsdaten empfangen werden können, angebracht und von ihr abgetrennt werden kann.

8. Vorrichtung (10) zur medizinischen Versorgung nach einem der Ansprüche 1 bis 7, wobei die Einheit zur Erfassung der Vorgeschichte innerhalb einer Bassin-Einheit (2) vorgesehen ist, die eine Bassin-Steuerungseinheit (16) enthält.

9. System zur medizinischen Versorgung, aufweisend:
die Vorrichtung (10) zur medizinischen Versorgung nach einem der Ansprüche 1 bis 8; und
eine Analysevorrichtung (20), die dafür konfiguriert ist, aus der Speichereinheit (18b) extrahierte Informationen über die Vorgeschichte zu analysieren.

10. System zur medizinischen Versorgung nach Anspruch 9, ferner aufweisend eine Anzeigevorrichtung (204a), die dafür konfiguriert ist, Informationen über die Vorgeschichte anzuzeigen, wobei
die Analysevorrichtung (20) dafür konfiguriert ist, zu veranlassen, dass die Anzeigevorrichtung (204a) die in der Vorrichtung (10) zur medizinischen Versorgung gespeicherten Informationen über die Vorgeschichte als Zeitreihendiagramm anzeigt.

11. System zur medizinischen Versorgung nach Anspruch 9 oder 10, ferner aufweisend eine Kommunikationseinheit (205), die dafür konfiguriert ist, die in der Vorrichtung (10) zur medizinischen Versorgung gespeicherten Informationen über die Vorgeschichte und/oder ein als Ergebnis einer mittels der Analysevorrichtung (20) durchgeführten Analyse erhaltenes Analyseergebnis an einen Server zu senden.

## Revendications

1. Appareil de soins médicaux (10) comprenant : une pluralité d'unités, la pluralité comprenant une unité d'entraînement de siège (11), et une unité de commande de fonctionnement (12),
la pluralité d'unités étant configurées pour communiquer entre elles et effectuer une pluralité de types d'opérations de commande ; et
une unité de collecte d'historiques (18) connectée à d'autres unités de commande (12, 14-17, 19) configurées pour communiquer avec les unités de commande (12, 14-17, 19), le dispositif de collecte d'historiques étant configuré pour collecter des données d'utilisation auprès de chacune des unités de commande (12, 14-17, 19),
l'unité de collecte d'historiques comportant
une unité de collecte (18) configurée pour collecter des données d'utilisation concernant chacun de la pluralité de types d'opérations de commande à partir de données de communication qui sont transmises et reçues parmi les unités de commande (12, 14-17, 19), et
une unité de stockage (18b) configurée pour stocker les données d'utilisation collectées par l'unité de collecte (18),
l'unité de collecte (18) étant configurée pour
collecter des données d'utilisation et amener l'unité de stockage (18b) à stocker des données d'utilisation collectées qui ont changé par rapport à des données d'utilisation actuellement stockées dans l'unité de stockage (18b).

2. Appareil de soins médicaux (10) selon la revendication 1, dans lequel l'unité de collecte (18) est configurée pour collecter des informations concernant une opération de commande par chacune des unités de commande (12, 14-17, 19) en tant que données d'utilisation à collecter.

3. Appareil de soins médicaux (10) selon la revendication 1, dans lequel l'unité de collecte (18) est configurée pour collecter, en tant que données d'utilisation à collecter, des informations concernant une opération de commande sélectionnée parmi la pluralité de types d'opérations de commande effectuées par les unités de commande (12, 14-17, 19).

4. Appareil de soins médicaux (10) selon l'une des revendications 1 à 3, dans lequel l'unité de collecte d'historiques comporte en outre une unité de sortie (18a) configurée pour délivrer en sortie les données d'utilisation stockées dans l'unité de stockage (18b).

5. Appareil de soins médicaux (10) selon l'une des revendications 1 à 3, dans lequel l'unité de stockage (18b) est un support de stockage qui peut être fixé au dispositif de collecte d'historiques et détaché de celui-ci.

6. Appareil de soins médicaux (10) selon la revendication 5, dans lequel l'unité de stockage (18b) est disposée à l'intérieur d'un boîtier incorporé dans l'appareil de soins médicaux (10) et a une porte (2d) qui peut être ouverte et fermée à l'aide d'un outil prescrit.

7. Appareil de soins médicaux (10) selon l'une des revendications 1 à 6, dans lequel l'unité de collecte d'historiques peut être fixée à une ligne de câblage, et détachée de celle-ci, à travers laquelle les données de communication transmises et reçues parmi les unités de commande (12, 14-17, 19) peuvent être reçues.

8. Appareil de soins médicaux (10) selon l'une des revendications 1 à 7, dans lequel l'unité de collecte d'historiques est disposée à l'intérieur d'une unité de lavabo (2) comportant une unité de commande de lavabo (16) .

9. Système de soins médicaux comprenant :
l'appareil de soins médicaux (10) selon l'une des revendications 1 à 8 ; et
un appareil d'analyse (20) configuré pour analyser des informations historiques extraites de l'unité de stockage (18b).

10. Système de soins médicaux selon la revendication 9, comprenant en outre un dispositif d'affichage (204a) configuré pour afficher des informations historiques, dans lequel
l'appareil d'analyse (20) est configuré pour amener le dispositif d'affichage (204a) à afficher, sous la forme d'un graphique en série chronologique, les informations historiques stockées dans l'appareil de soins médicaux (10).

11. Système de soins médicaux selon la revendication 9 ou 10, comprenant en outre une unité de communication (205) configurée pour transmettre, à un serveur, au moins l'un parmi : des informations historiques stockées dans l'appareil de soins médicaux (10) ; et un résultat d'analyse obtenu en résultat d'une analyse effectuée par l'appareil d'analyse (20).
